(19) Europäisches Patentamt
European Patent Office
Office européen des brevets

(11) Veröffentlichungsnummer: **0 017 99**
**A2**

# (12) EUROPÄISCHE PATENTANMELDUNG

(21) Anmeldenummer: **80102090.0**

(22) Anmeldetag: **18.04.80**

(51) Int. Cl.³: **C 07 D 209/52**, C 07 D 307/93, A 01 N 43/38

(30) Priorität: **20.04.79 CH 3748/79**
**31.03.80 CH 2539/80**

(43) Veröffentlichungstag der Anmeldung: **29.10.80**
**Patentblatt 80/22**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Bühler, Niklaus, Dr., Gartenweg 30, CH-4310 Rheinfelden (CH)**
Erfinder: **Baumann, Marcus, Dr., Rührbergerstrasse 6, CH-4058 Basel (CH)**
Erfinder: **Bellus, Daniel, Dr., Unterm Schellenberg 81, CH-4125 Riehen (CH)**
Erfinder: **Sturm, Elmar, Dr., Ziegelbündtenweg 20 B, CH-4147 Aesch (CH)**

(74) Vertreter: **Zumstein sen., Fritz, Dr. et al, Bräuhausstrasse 4, D-8000 München 2 (DE)**

(54) **Cyclobutandicarbonsäureimide, Verfahren zu deren Herstellung und deren Verwendung als Fungizid; Zwischenprodukte.**

(57) Cyclobutandicarbonsäureimide der Formel

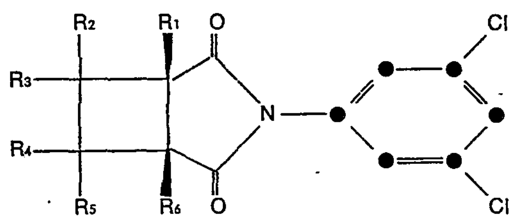

(I),

worin eines von $R_1$ und $R_6$ Wasserstoff, Methyl oder Äthyl und das andere Methyl oder Äthyl bedeutet, $R_2$ und $R_3$ unabhängig voneinander Wasserstoff, Fluor oder Methyl darstellen oder aber $R_2$ Acetoxy oder Chlor und gleichzeitig $R_3$ Wasserstoff bedeutet; $R_4$ Wasserstoff, Methyl oder Chlor ist und $R_5$ Wasserstoff oder Methyl darstellt, wobei auch $R_3$ und $R_4$ zusammen eine zusätzliche Bindung im Vierring bilden können, eignen sich zur Bekämpfung verschiedenartiger Pilze und besitzen insbesondere eine ausgezeichnete Botrytis-Wirkung. Sie lassen sich auf besonders vorteilhafte Weise durch photochemische [2+2] Cycloaddition von Alkenen oder Alkinen an z. B. entsprechend substituierten Maleinsäure-(3,5-dichlorphenyl)-imide herstellen.

EP 0 017 994 A2

0017994



CIBA-GEIGY AG
Basel (Schweiz)

5-12324/1+2/ZFO

BEZEICHNUNG GEÄNDERT
siehe Titelseite

Neue Cyclobutandicarbonsäureimide, Verfahren zu deren Herstellung und deren Verwendung als Fungizid

Die vorliegende Erfindung betrifft neue Cyclobutandicarbonsäureimide, Verfahren zu deren Herstellung, fungizide Mittel, die solche Verbindungen als Aktivsubstanz enthalten sowie die Verwendung dieser Verbindungen zur Bekämpfung von Pilzen.

Aus der japanischen Offenlegungsschrift 74/71 141 ist bekannt, dass am Cyclobutanring unsubstituierte 3,5-Dihalogenphenylcyclobutandicarbonsäureimide antimikrobielle, u.a. auch fungizide Eigenschaften aufweisen und sich beispielsweise zur Bekämpfung von Pilzerkrankungen am Reis eignen. Das Wirkungsspektrum dieser 3,5-Dihalogenphenylcyclobutandicarbonsäureimide ist jedoch beschränkt. So zeigt z.B. das 3,5-Dichlorphenylcyclobutandicarbonsäureimid nur eine schwache Botrytis-Wirkung.

Es hat sich gezeigt, dass die erfindungsgemässen Verbindungen der Formel I ausgezeichnete fungizide Eigenschaften aufweisen,

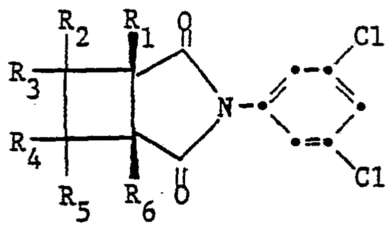

(I),

worin eines von $R_1$ und $R_6$ Wasserstoff, Methyl oder Aethyl und das andere Methyl oder Aethyl bedeutet, $R_2$ und $R_3$ unabhängig voneinander Wasserstoff, Fluor oder Methyl darstellen oder aber $R_2$ Acetoxy oder Chlor und gleichzeitig $R_3$ Wasserstoff bedeutet; und $R_4$ Wasserstoff Methyl oder Chlor ist und $R_5$ Wasserstoff oder Methyl darstellt, wobei auch $R_3$ und $R_4$ zusammen eine zusätzliche Bindung im Vierring bilden können.

Verbindungen der Formel I besitzen überraschenderweise eine ausgeprägte Botrytis-Wirkung. Botrytis spp. (B.cinerea, B.allii) stellen mit der Grauschimmelfäule an Reben, Erdbeeren, Aepfeln, Zwiebeln und anderen Obst- und Gemüsesorten einen bedeutenden wirtschaftlichen Schadfaktor dar.

Die Verbindungen der Formel I besitzen ein für die praktischen Bedürfnisse sehr günstiges Wirkungsspektrum zum Schutze von Kulturpflanzen, ohne diese durch unerwünschte Nebenwirkungen nachteilig zu beeinflussen. Kulturpflanzen seien im Rahmen vorliegender Erfindung beispielsweise Getreide, Mais, Reis, Gemüse, Zuckerrüben, Soja, Erdnüsse, Obstbäume, Zierpflanzen, Reben, Hopfen, Gurkengewächse (Gurken, Kürbis, Melonen), Solanaceen, wie Kartoffeln, Tomaten und Tabak, sowie auch Bananen-, Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilze wirksam: Ascomycetes (z.B. Erysiphaceae, Fusarium, Helminthosporium); Basidiomycetes, wie vor allem Rostpilze (z.B. Puccinia, Tilletia);

Fungi imperfecti (z.B. Moniliales u.a., Cercospora, Sclerotinia sowie Botrytis und Piricularia) und die der Klasse der Phycomycetes angehörenden Oomycetes, wie Phytophthora oder Plasmopara. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Eine wichtige Gruppe von Aktivsubstanzen sind jene Verbindungen der Formel I, worin $R_3$ und $R_4$ eine zusätzliche Bindung bilden. Es sind also Verbindungen der Formel I, die einen Cyclobutenring enthalten.

Eine weitere Gruppe wichtiger Aktivsubstanzen sind solche Verbindungen der Formel I, worin eines von $R_1$ und $R_6$ Wasserstoff, Methyl oder Aethyl und das andere Methyl oder Aethyl ist, $R_2$ und $R_3$ unabhängig voneinander Wasserstoff, Fluor oder Methyl darstellen und $R_4$ und $R_5$ unabhängig voneinander Wasserstoff oder Methyl bedeuten.

Bevorzugt sind Verbindungen der Formel I als Aktivsubstanzen, worin $R_1=R_6$ ist und $R_4=R_5$, worin also $R_1$ und $R_6$ gleiche Bedeutung haben und Methyl oder Aethyl darstellen, $R_2$ und $R_3$ unabhängig voneinander Wasserstoff, Fluor oder Methyl bedeuten und $R_4$ und $R_5$ gleiche Bedeutung haben und Wasserstoff oder Methyl darstellen.

Besonders bevorzugt sind Verbindungen der Formel I, worin $R_1$ und $R_6$ je Methyl, $R_2$ und $R_3$ unabhängig voneinander Wasserstoff oder Fluor und $R_4$ und $R_5$ je Wasserstoff darstellen. Zu den besonders bevorzugten Einzelverbindungen gehören die weiter unten genannten Verbindungen Nr.1, 2, 7, 9, 15.

Die Verbindungen der Formel I lassen sich nach einem einfachen und wirtschaftlichen Verfahren in guten Ausbeuten dadurch herstellen, dass man

a) eine Verbindung der Formel II

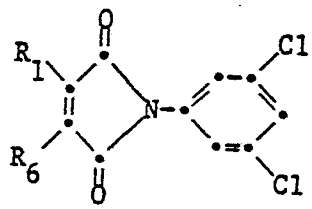

(II)

vorzugsweise in Gegenwart eines Sensibilisators durch Lichteinwirkung bei einer Wellenlänge kürzer als 5000 Ångström (Å) mit einer Verbindung der Formel III

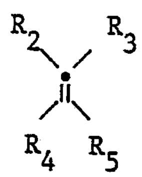

(III)

umsetzt oder

b) die Verbindung der Formel III schrittweise unter gleichen Reaktionsbedingungen einleitend mit einer Verbindung der Formel IV

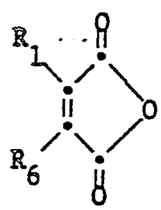

(IV)

zu einer Verbindung der Formel V

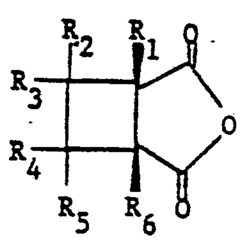

(V)

umsetzt, diese mit 3,5-Dichloranilin reagieren lässt und die dabei entstehende Amidcarbonsäure der Formel VI

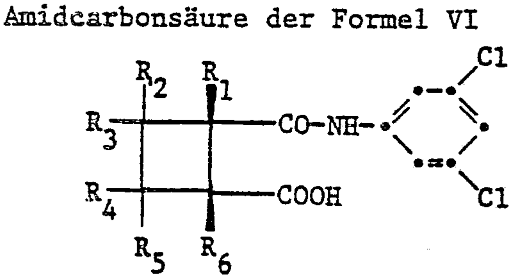

(VI)

anschliessend cyclisiert, wobei $R_1$ bis $R_6$ in den obigen Formeln II bis VI die unter Formel I angegebene Bedeutung haben. Bevorzugt ist die Herstellung gemäss Verfahrensvariante a).

Die erfindungsgemässe photochemische [2+2] Cycloaddition von Verbindungen der Formel III an Verbindungen der Formel II bzw. IV ist insbesondere auch vom ökologischen Standpunkt aus günstig. Die Reaktion verläuft überraschenderweise ohne Bildung von Dimeren der Maleinsäureanhydride bzw. -imide, wie sie sonst bei vergleichbaren photochemischen Cycloadditionen stets auftreten [vgl. z.B. Chem.Ber., 110, 2986-2995 (1977); Chem.Ber., 96, 498-508 (1962) und Chem.Ber., 98, 764-780 (1965); insbesondere Zhurnal Organicheskoi Khimii, Vol.8, No.2, pp.377-382 (Feb.1972)].

Die photochemische [2+2]-Cycloaddition der Verbindungen der Formel III an Verbindungen der Formel II oder IV wird mit Vorteil in Gegenwart eines inerten organischen Lösungsmittels bei Temperaturen zwischen etwa -80°C und +30°C, bevorzugt etwa -78° und -20°C, durchgeführt.

Geeignete inerte organische Lösungsmittel sind z.B. gegebenenfalls chlorierte aliphatische oder aromatische Kohlenwasserstoffe, wie Dichlormethan, 1,2-Dichloräthan, 1,2,3-Trichlorpropan, 1,1,2,2-Tetrachloräthan, Benzol, Chlorbenzol und Dichlorbenzole; aliphatische Carbonsäureester, wie Essigsäuremethylester und Essigsäureäthylester, Dialkyläther mit je 2-6 C-Atomen in den Alkylteilen, wie Diäthyläther, Di-n-propyläther und Di-isopropyläther; cyclische Aether, wie Dioxan und Tetrahydrofuran; Alkylnitrile, besonders solche mit 2-5 C-Atomen, wie Acetonitril, Propionitril und Butyronitril. Bevorzugt als Lösungsmittel sind chlorierte aliphatische Kohlenwasserstoffe, besonders Dichlormethan. Auch Aceton oder Methyläthylketon kommen in Frage.

Als Sensibilisatoren für die oben erwähnten photochemischen Cycloadditionen können an sich bekannte Verbindungen eingesetzt werden, bevorzugt solche, die eine $E_T$ (Triplett-Energie) von $\geq$230 kJ/mol aufweisen [vgl. Paul S. Engel und Bruce M. Monroe "Advance in Photochemistry, Bd. 8, 297-306, New York 1971]. Beispiele derartiger Sensibilisatoren sind Benzophenon, Acetophenon, m-Methoxy-acetophenon, Thioxanthon und Biacetyl. Vorzugsweise verwendet man Benzophenon als Sensibilisator. Im allgemeinen wird der Sensibilisator in einer Menge von etwa 0,5-10 Gew.%, bezogen auf die Ausgangsverbindung der Formel II oder IV, eingesetzt.

Für die erfindungsgemässen photochemischen Umsetzungen können Lichtquellen eingesetzt werden, die Licht mit Wellenlängen unter 5000 Å emittieren, wie z.B. gegebenenfalls metallatomdotierte Quecksilberhochdrucklampen, Xenondampflampen, Quecksilber-Xenonlampen, Quecksilberniederdruck- oder -mitteldrucklampen, Halogenlampen oder $D_2$-Lampen.

Die photochemischen Reaktionen werden zweckmässig in einer Tieftemperaturbelichtungsanlage vorgenommen. Dabei wird eine etwa 2-20%ige Lösung der Verbindung der Formel II oder IV zusammen mit dem Sensibilisator an die Belichtungsanlage gegeben, dann über einen Gaseinlass mit der gewünschten Verbindung der Formel III gesättigt und anschliessend, abhängig von Konzentration, Quantenausbeute und Photonenflux, während etwa 2-10 Stunden belichtet. Die Aufarbeitung des Reaktionsproduktes erfolgt z.B. durch Einengen der Reaktionslösung und Umkristallisation des Rohproduktes aus geeigneten organischen Lösungsmitteln, bevorzugt Gemischen von Dichlormethan und n-Hexan. Die erhaltenen Produkte der Formel I bzw. V sind analysenrein.

Die Umsetzung der Anhydride der Formel V mit dem 3,5-Dichloranilin wird auf an sich bekannte Weise vorgenommen, zweckmässig ohne Lösungsmittel bei Temperaturen zwischen etwa 80° und 120°C oder in organischem Medium bei Temperaturen zwischen etwa 20° und 120°C. Als organische Lösungsmittel eignen sich z.B. gegebenenfalls chlorierte aliphatische oder aromatische Kohlenwasserstoffe, wie Benzol, Toluol, Xylol, Dichlormethan, Chloroform, Tetrachlorkohlenstoff, o-Dichlorbenzol; aliphatische oder cycloaliphatische Ketone, wie Aceton, Methyläthylketon, Cyclopentanon und Cyclohexanon; ferner Dialkyläther mit je 2-6 C-Atomen in den Alkylteilen und cyclische Aether, wie Tetrahydrofuran und Dioxan.

Die Cyclisierung der Amidcarbonsäuren der Formel VI unter Wasserabspaltung erfolgt ebenfalls nach üblichen Methoden thermisch, d.h. durch blosses Erhitzen des Reaktionsgemisches, oder unter Verwendung an sich bekannter Dehydratisierungsmittel bei Temperaturen zwischen etwa 20° und 120°C. Als Dehydratisierungsmittel kommen vor allem Anhydride von gegebenenfalls durch Halogenatome oder Alkylgruppen substituierten aliphatischen Monocarbonsäuren mit 2-5 C-Atomen in Betracht, wie Essigsäure-, Propionsäure-, Buttersäure- und Valeriansäureanhydrid, Trichlor-, Trifluor-, Trimethyl-, Triäthyl- und Tri-n-butyl-essigsäureanhydrid. Bevorzugtes Dehydratisierungsmittel ist Essigsäureanhydrid.

Die Umsetzung der Anhydride der Formel I mit dem 3,5-Dichloranilin kann auch ohne Zusatz von Lösungsmitteln vorgenommen werden, wobei die dabei entstehenden Amidcarbonsäuren zweckmässig thermisch, d.h. durch trockenes Erhitzen, cyclisiert werden.

Die Ausgangsverbindungen der Formeln II, III, IV und das 3,5-Dichloranilin sind bekannt oder können nach an sich bekannten Methoden hergestellt werden. Verbindungen der Formel II können z.B. durch Umsetzung von Anhydriden der Formel IV mit 3,5-Dichloranilin erhalten werden.

Gegenstand der Erfindung sind auch die neuen Zwischenprodukte der Formel VI sowie die Zwischenprodukte der Formel V, insbesondere Verbindungen der Formel V, worin $R_1$ und $R_6$ gleiche Bedeutung haben und Methyl oder Aethyl darstellen und $R_2$ bis $R_5$ die unter Formel I angegebene Bedeutung besitzen.

Herstellungsbeispiele

Beispiel 1: In einer Tieftemperaturbelichtungsanlage, ausgerüstet mit einem 125 Watt Quecksilberhochdruckbrenner in gekühltem Pyrex-Tauchschacht, Rührer und Gaseinleitrohr, werden 10 g (0,0 37 Mol) 2,3-Dimethylmaleinsäure-(3,5-dichlorphenyl)-imid und 1,0 g (0,0055 Mol) Benzophenon in 220 ml Dichlormethan eingebracht und durch ein externes Kühlbad mit einer Aceton-Trockeneis-Mischun auf -50 bis-55°C abgekühlt. Diese Lösung wird nun während 2 Stunden mit Vinyl-fluorid begast und anschliessend unter schwachem Vinylfluoridstrom 6 Stunden belichtet. Sodann wird das Lösungsmittel abgedampft. Man erhält 4,8 g weisse Kristalle. Die Umkristallisation aus Dichlormethan/n-Hexan liefert 4,0 g (47% d.Th.) 1,2-Dimethyl-3-fluor-cyclobutan-1,2-dicarbonsäure-(3,5-dichlorphenyl)-imid in Form weisser Kristalle; Smp. 122-123°C. NMR-Spektrum (100 MHz, $CDCl_3$, $\delta$ in ppm): 1,4 (m, 6H, $2CH_3$); 2,0-3,0 (m, 2H, $-CH_2-$); 4,6-5,4(2m, -CHF-); 7,2-7,4 (m, 3H, aromat.). IR-Spektrum (KBr, $cm^{-1}$, vs = sehr stark, s = stark, m = mittel, w = schwach): $1700^{vs}$ , $1590^{m}$, $1585^{s}$, $1450^{s}$, $1360^{s}$, $1150^{s}$, $805^{s}$. Verbindung Nr. 2.

Beispiele 2-6: Auf analoge Weise wie in Beispiel 1 beschrieben werden hergestel

- 1,2-Dimethyl-cyclobutan-1,2-dicarbonsäure-(3,5-dichlorphenyl)-imid; Smp. 149-152°C, ausgehend von 2,3-Dimethylmaleinsäure-(3,5-dichlorphenyl)-imi und Aethylen; Verbindung Nr.1.
- 1,2,3-Trimethyl-cyclobutan-1,2-dicarbonsäure-(3,5-dichlorphenyl)-imid; Smp. 88-89°C, ausgehend von 2,3-Dimethylmaleinsäure-(3,5-dichlorphenyl)-imid und 1-Propen; Verbindung Nr.3
- 3,3-Difluor-1,2-dimethyl-cyclobutan-1,2-dicarbonsäure-(3,5-dichlorphenyl)-imid; Smp. 135-140°C, ausgehend von 2,3-Dimethylmaleinsäure-(3,5-dichlorphenyl)-imid und 1,1-Difluoräthylen; Verbindung Nr.4.

- 2-Aethyl-1-methyl-cyclobutan-1,2-dicarbonsäure-(3,5-dichlorphenyl)-imid; Smp. 91-93°C, ausgehend von 2-Aethyl-3-methylmaleinsäure-(3,5-dichlorphenyl)-imid und Aethylen; Verbindung Nr. 5.
- 1,2,3,4-Tetramethyl-cyclobutan-1,2-dicarbonsäure-(3,5-dichlorphenyl)-imid; Smp. 81-83°C, ausgehend von 2,3-Dimethylmaleinsäure-(3,5-dichlorphenyl)-imid und trans-2-Buten; Verbindung Nr. 6.

Beispiel 7: a) In einer Tieftemperaturbelichtungsanlage, ausgerüstet mit einem 125 W Quecksilberhochdruckbrenner in gekühltem Tauchbad, Rührer und Gaseinleitungsrohr, werden 15 g (0,12 Mol) Dimethylmaleinsäureanhydrid und 1 g Benzophenon (0,0054 Mol) in 300 ml Dichlormethan gelöst und durch ein externes Kühlbad mit einer Isopropanol-Trockeneis-Mischung auf -60 bis -70°C abgekühlt, eine Stunde mit Aethylen begast und anschliessend bei dieser Temperatur 12 Stunden unter schwachem Aethylenstrom bestrahlt. Sodann wird das Lösungsmittel abgedampft, und das Rohprodukt wird aus Dichlormethan/n-Hexan umkristallisiert. Man erhält 10 g 1,2-Dimethyl-cyclobutan-1,2-dicarbonsäureanhydrid (54% d.Th.) in Form weisser Kristalle; Smp. 84-86°C. NMR-Spektrum (100 MHz), $\delta$ in ppm: 1,4 (s, 6H, $2CH_3$); 2,0-2,5 (m, 4H, $-CH_2CH_2-$). IR-Spektrum (KBr, $cm^{-1}$, vs = sehr stark, s = stark, m = mittel, w = schwach): $2940^m$, $1850^s$, $1780^{vs}$, $1470^m$, $1445^m$, $1300^s$, $1280^s$, $1240^s$, $1170^{vs}$, $970^{vs}$, $950^{vs}$, $920^{vs}$.

b) 15,4 g (0,1 Mol) 1,2-Dimethyl-cyclobutan-1,2-dicarbonsäureanhydrid werden in 100 ml Toluol gelöst und mit 16,2 g (0,1 Mol) 3,5-Dichloranilin versetzt. Nach 16-stündigem Rühren bei 25°C wird die ausgefallene farblose Amidcarbonsäure abgetrennt. Man erhält 27,8 g 1-(3,5-Dichlorphenylcarbamoyl)-1,2-dimethylcyclobutan-1-carbonsäure (88% d.Th.) vom Smp. 164°C. NMR-Spektrum (60 MHz) $\delta$ in ppm: 1,34 (s, 3H, $CH_3$); 1,45 (s, 3H, $CH_3$); 1,6-3,0 (m, 4H, $-CH_2CH_2-$); 6,9 (m, 1H, aromat.); 7,6 (m, 2H, aromat.); 8,75 (s, 1H, -COOH, mit $D_2O$ austauschbar).

Elementaranalyse für $C_{14}H_{15}Cl_2NO_3$ (Molgewicht 316,2):

| | | | | | |
|---|---|---|---|---|---|
| berechnet: | C 53,2% | H 4,8% | N 4,4% | O 15,2% | Cl 22,4% |
| gefunden: | C 53,2% | H 4,8% | N 4,7% | O 15,2% | Cl 22,5%. |

$C_1$) 14,3 g der obigen Amidcarbonsäure werden in 100 ml Essigsäureanhydrid 6 Stunden am Rückfluss gekocht. Nach dem Abkühlen wird das ausgefallene Imid abfiltriert. Man erhält 10,3 g (77% d.Th.) 1,2-Dimethyl-cyclobutan-1,2-dicarbonsäure-(3,5-dichlorphenyl)-imid, dessen Eigenschaften mit denjenigen der gemäss Beispiel 1 hergestellten Verbindung übereinstimmen.

$C_2$) Die Cyclisierung der Amidcarbonsäure kann auch dadurch erfolgen, dass man 3,2 g (0,01 Mol) 1-(3,5-Dichlorphenylcarbamoyl)-1,2-dimethyl-cyclobutan-1-carbonsäure 2 Stunden auf 160°C erhitzt. Nach dem Abkühlen wird aus Essigsäureäthylester umkristallisiert. Man erhält 1,3 g (44% d.Th.) 1,2-Dimethyl-cyclobutan-1,2-dicarbonsäure-(3,5-dichlorphenyl)-imid.

Das 1,2-Dimethyl-cyclobutan-1,2-dicarbonsäure-(3,5-dichlorphenyl)-imid kann auch wie folgt hergestellt werden:
$C_3$)7,7 g (0,05 Mol) 1,2-Dimethylcyclobutan-1,2-dicarbonsäureanhydrid werden mit 8,1 g (0,05 Mol) 3,5-Dichloranilin 8 Stunden auf 180°C erwärmt. Nach dem Abkühlen wird aus Essigsäureäthylester umkristallisiert. Man erhält 5,1 g (34% d.Th.) 1,2-Dimethylcyclobutan-1,2-dicarbonsäure-(3,5-dichlorphenyl)-imid, dessen Eigenschaften mit denjenigen der gemäss Beispiel 1 hergestellten Verbindung identisch sind.
$C_4$)7,7 g (0,05 Mol) 1,2-Dimethylcyclobutan-1,2-dicarbonsäureanhydrid und 8,1 g (0,05 Mol) 3,5-Dichloranilin werden in 100 ml Xylol gelöst und 8 Stunden mit einem Wasserabscheider am Rückfluss gekocht. Anschliessend wird abgekühlt, und das ausgefallene Imid wird abfiltriert. Man erhält 11,6 g (78% d.Th.) 1,2-Dimethylcyclobutan-1,2-dicarbonsäure-(3,5-dichlorphenyl)-imid.

<u>Beispiel 8</u>: a) Analog der im Beispiel 7a beschrieben Arbeitsweise wird das 1-Methyl-cyclobutan-1,2-dicarbonsäureanhydrid (Smp. 59-60°C) durch Umsetzung von Citraconsäureanhydrid mit Aethylen hergestellt.

b) 10 g (0,07 Mol) 1-Methyl-cyclobutan-1,2-dicarbonsäureanhydrid werden in 50 ml Toluol gelöst und mit 11,6 g (0,07 Mol) 3,5-Dichloranilin versetzt. Nach 24-stündigem Rühren bei Raumtemperatur wird die ausgefallene Amidsäure (Smp. 106-108°C) abgetrennt. Diese Amidcarbonsäure wird in 40 ml Essigsäureanhydrid aufgeschlämmt und 24 Stunden bei Raumtemperatur gerührt, wobei alles in Lösung geht. Das Essigsäureanhydrid wird am Wasserstrahlvakuum abgezogen, und der pulverige Rückstand wird aus Dichlormethan/n-Hexan umkristallisiert. Man erhält 15 g (73% d.Th.) 1-Methyl-cyclobutan-1,2-dicarbonsäure (3,5-dichlorphenyl)-imid; Smp. 110-111°C. NMR-Spektrum (100 MHz) δ in ppm): 1,55 (s, 3H, $CH_3$); 2,0-3,2 (m, 5H, $-CH_2CH_2-CH-$); 7,2-7,5 (m, 3H, aromat.).

Verbindung Nr.7.

Analog dem Beispiel 8 werden auch folgende Verbindungen der Formel I hergestellt: ($R_5$=H)

| Verb. | $R_1$ | $R_6$ | $R_2$ | $R_3$ | $R_4$ | Smp.°C |
|---|---|---|---|---|---|---|
| 8 | $CH_3$ | H | *$CH_3$[H] | H | *H[$CH_3$] | 123-128° |
| 9 | $CH_3$ | H | *F[H] | H | *H[F] | 106-109° |
| 10 | $CH_3$ | $CH_3$ | Cl | H | H | 140-143° |
| 11 | $CH_3$ | $CH_3$ | Cl | H | Cl | 192-197° |
| 12 | $CH_3$ | $CH_3$ | $CH_3$ | $CH_3$ | H | 96-98° |
| 13 | $CH_3$ | H | F | F | H | 176-178° |
| 14 | $CH_3$ | $CH_3$ | $CH_3COO$ | H | H | 122-124° |

* Stellungsisomerie bei $R_2$ und $R_4$

Beispiel 9

Herstellung von

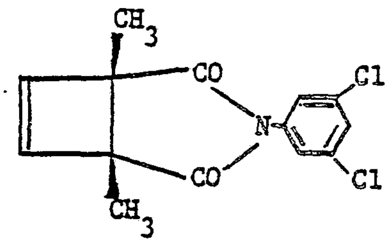

1,2-Dimethyl-cyclobut-3-en-1,2-dicarbonsäure-(3,5-dichlorphenyl)-imid [Verbindung Nr.15].

a) Gemäss dem Beispiel 7a werden 10 g (0,079 Mol) 2,3-Dimethylmaleinsäureanhydrid und 1 g (0,0054 Mol) Benzophenon in 250 ml Dichlormethan bei -60°C eine Stunde mit Acetylen begast und anschliessend 6 Stunden unter schwachem Acetylenstrom belichtet. Nach Abdampfen des Lösungsmittels und Umkristallisation aus Dichlormethan/Hexan erhält man 7,5 g (62% der Theorie) 1,2-Dimethyl-cyclobut-3-en-1,2-dicarbonsäureanhydrid, Smp. 84-86°C.

b) 6,9 g (0,0454 Mol) des gemäss a) hergestellten Zwischenprodukts werden in 50 ml Toluol zusammen mit 7,4 g (0,0454 Mol) 3,5-Dichloranilin 24 Stunden bei Raumtemperatur gerührt. Der erhaltene weisse Niederschlag wird in 50 ml Acetanhydrid 48 Stunden bei Raumtemperatur und 8 Stunden bei 40°C gerührt. Das nach dem Einengen erhaltene Oel wird aus Dichlormethan/Hexan umkristallisiert:
3,6 g (27% d.Th.) Endprodukt, Smp. 158-160°C.

Entsprechend werden die folgenden Verbindungen der Formel I erhalten, bei denen $R_3$ und $R_4$ eine zusätzliche Bindung im Vierring bilden:

| Verbindung | $R_1$ | $R_6$ | $R_2$ | $R_5$ | Smp. °C |
|---|---|---|---|---|---|
| 16 | $CH_3$ | $CH_3$ | $CH_3$ | H | 142-143 ° |
| 17 | $CH_3$ | $C_2H_5$ | H | H | 137-140° |
| 18 | $CH_3$. | H | *$CH_3$[H] | *H[$CH_3$] | 129-135° |

* Stellungsisomerie

Grundsätzlich lässt sich jede der Verbindungen der Formel I durch photochemische Reaktion sowohl mit dem gewünschten Imid, wie auch mit dem gewünschten Anhydrid (und anschliessende Weiterreaktion mit 3,5-Dichloranilin) herstellen, d.h. nach jedem der weiter oben geschilderten Verfahrensvarianten a) und b), wie sie in den Beispielen 1 bis 9 illustriert werden.

Die Verbindungen der Formel I können für sich ellein oder zusammen mit geeigneten Träger und/oder anderen Zuschlagstoffen verwendet werden. Geeignete Träger und Zuschlagsstoffe können fest oder flüssig sein und entsprechen den in der Formulierungstechnik üblichen Stoffen wie z.B. natürlichen oder regenerierten mineralischen Stoffen, Lösungs-Dispergier-, Netz-, Haft-, Verdickungs-, Binde- oder Düngemitteln. Wirkstoffe der Formel I können im Gemisch mit anderen pestiziden oder pflanzenwuchsverbessernden Präparaten verwendet werden.

Der Gehalt an Wirkstoff in handelsfähigen Mitteln liegt zwischen 0,1 bis 90%.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel (bis zu 10%); Granulate, Umhüllungsgranulate, Imprägnierungsgranulate und Homogengranulate, Pellets (Körner) (1 bis 80%);

Flüssige Aufarbeitungsformen:

a) in Wasser dispergierbare Wirkstoffkonzentrate:
   Spritzpulver (wettable powders) und Pasten (25-90% in der Handelspackung, 0,01 bis 15% in gebrauchsfertiger Lösung); Emulsions- und Lösungskonzentrate (10 bis 50%; 0,01 bis 15% in gebrauchsfertiger Lösung);

b) Lösungen (0,1 bis 20%); Aerosole.

Die Wirkstoffe der Formel I vorliegender Erfindung können beispielsweise wie folgt formuliert werden:

Spritzpulver: Zur Herstellung eines a) 70%igen b) 40%igen c) 25%igen d) 10%igen Spritzpulvers werden folgende Bestandteile verwendet:

a) 70 Teile Wirkstoff
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin
12 Teile Champagne-Kreide;

b) 40 Teile Wirkstoff
5 Teile Ligninsulfonsäure-Natriumsalz,
1 Teil Dibutylnaphthalinsulfonsäure-Natriumsalz,
54 Teile Kieselsäure;

c) 25 Teile Wirkstoff
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

d) 10 Teile Wirkstoff
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Emulgierbare Konzentrate: Zur Herstellung eines 25%igen emulgierbaren Konzentrates werden folgende Stoffe verwendet:

25 Teile Wirkstoff
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Konzentration hergestellt werden, die besonders zur Blattapplikation geeignet sind.

## Biologische Beispiele

a) Wirkung gegen Botrytis cinerea auf Bohnen

Residual-protektive Wirkung: Ca. 10 cm hohe Bohnen-Pflanzen wurden mit einer aus Spritzpulver des Wirkstoffes hergestellten Spritzbrühe (0,006% bzw. 0,002% Aktivsubstanz) besprüht. Nach 48 Stunden wurden die behandelten Pflanzen mit einer Konidiensuspension des Pilzes infiziert. Nach einer Inkubation der infizierten Pflanzen während 2-3 Tagen bei 95-100% relativer Luftfeuchtigkeit und 21°C erfolgte die Beurteilung des Pilzbefalls. Bei einer Anwendungskonzentration von 0,006% bewirkten sämtliche Verbindungen der Formel I, nämlich die Verbindungen Nr.1 bis 18, eine vollständige Verhütung des Krankheitsbefalls (0-5% Pilzbefall). Bei einer Anwendungskonzentration von 0,002% wurde der Krankheitsbefall mit den Verbindungen Nr.1, 2, 5, 7, 9, 10, 15 und 16 vollständig verhütet (0-5% Pilzbefall), mit den übrigen der 18 aufgeführten Verbindungen stark zurückgedrängt (5-20% Pilzbefall).

b) Wirkung gegen Sclerotinia fructigena auf Aepfeln

Künstlich verletzte Aepfel wurden mit Sclerotinia fructigena infiziert, indem auf jede Verletzungsstelle ein Tropfen Myzelsuspension pipettiert wurde. Nach Abtrocknen des Inokulum-Tropfens wurden die Aepfel mittels einer Spritzpulversuspension der Wirksubstanz (0,02% Aktivsubstanz) besprüht. Die behandelten Früchte wurden in Plastikbehälter gelegt und während 14 Tagen bei 20-22°C gelagert. Zur Auswertung wurde die Anzahl der angefaulten Verletzungsstellen bestimmt. Mit den Verbindungen Nr.1, 5, 7, 9, 10, 15 und 18 wurde der Krankheitsbefall fast vollständig verhütet (unter 10% Befall). Durch Behandlung mit der Verbindung Nr. 2 trat überhaupt kein Krankheitsbefall auf.

c) Wirkung gegen Erysiphe graminis auf Gerste. Residual protektive Wirkung:

ca.8 cm hohe Gerstenpflanzen wurden mit einer aus Spritzpulver des Wirkstoffes hergestellten Spritzbrühe (0,02% Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt. Neben anderen Verbindungen wurde mit den Verbindungen Nr.11, 12, 14 und 16 der Pilzbefall vollständig verhütet.

d) Wirkung gegen Monilinia auf Kirschblüten oder Pfirsichblüten (Monilinia-Befall an Steinobst)

Einzelne voll erblühte Steinobstzweige werden mit einer aus einem Emulsionskonzentrat hergestellten Spritzbrühe (enthaltend 0,025% Aktivsubstanz) besprüht. Einige Stunden später werden die Blütenstände abgeschnitten, mit dem Stiel in den nassen Stand von Plastikschalen gesteckt und mit einer Sporensuspension inokuliert. Die Schalen werden dann lose mit transparenter Plastikfolie bedeckt und 2 Tage bei Raumtemperatur gehalten. Mit der Zahl von befallenen Blüten wird das Ausmass des Krankheitsbefalls bestimmt, wobei je 40 Blüten pro Wirkstoff verwendet werden. Die Verbindungen Nr.1 bis 18 bewirken eine Reduktion des Krankheitsbefalls auf unter 20%. Einige Verbindungen verhüteten den Befall vollständig, z.B. Verb.Nr.2 und Nr.7.

Patentansprüche

1. Eine Verbindung der Formel

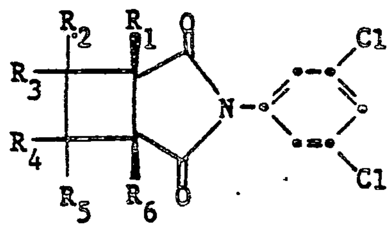

(I),

worin eines von $R_1$ und $R_6$ Wasserstoff, Methyl, oder Aethyl und das andere Methyl oder Aethyl bedeutet, $R_2$ und $R_3$, unabhängig voneinander Wasserstoff Fluor oder Methyl darstellen oder aber $R_2$ Acetoxy oder Chlor und gleichzeitig $R_3$ Wasserstoff bedeutet; $R_4$ Wasserstoff, Methyl oder Chlor ist und $R_5$ Wasserstoff oder Methyl darstellt, wobei auch $R_3$ und $R_4$ zusammen eine zusätzliche Bindung im Vierring bilden können.

2. Eine Verbindung der Formel I nach Anspruch 1, worin $R_3$ und $R_4$ eine zusätzliche Bindung bilden.

3. Eine Verbindung der Formel I nach Anspruch 1, worin eines von $R_1$ und $R_6$ Wasserstoff, Methyl oder Aethyl und das andere Methyl oder Aethyl ist, $R_2$ und $R_3$ unabhängig voneinander Wasserstoff, Fluor oder Methyl darstellen und $R_4$ und $R_5$ unabhängig voneinander Wasserstoff oder Methyl bedeuten.

4. Eine Verbindung nach Anspruch 4, worin $R_1$ und $R_6$ gleiche Bedeutung haben und Methyl oder Aethyl darstellen, $R_2$ und $R_3$ unabhängig voneinander Wasserstoff, Fluor oder Methyl darstellen und $R_4$ und $R_5$ gleiche Bedeutung haben und Wasserstoff oder Methyl darstellen.

5. Eine Verbindung der Formel I nach Anspruch 1, worin $R_1$ und $R_6$ je Methyl, $R_2$ und $R_3$ unabhängig voneinander Wasserstoff oder Fluor, $R_4$ und $R_5$ je Wasserstoff darstellen.

6. 1,2-Dimethyl-cyclobutan-1,2-dicarbonsäure-(3,5-dichlorphenyl)-imid nach Anspruch 1.

0017994

7. 1,2-Dimethyl-3-fluor-cyclobutan-1,2-dicarbonsäure-(3,5-dichlor-phenyl)-imid nach Anspruch 1.

8. 1-Methyl-cyclobutan-1,2-dicarbonsäure-(3,5-dichlorphenyl)-imid nach Anspruch 1.

9. 1,2-Dimethyl-cyclobut-3-en-1,2-dicarbonsäure-(3,5-dichlorphenyl)-imid nach Anspruch 1.

10. 1,2-Dimethyl-3-chlorcyclobutan-1,2-dicarbonsäure-(3,5-dichlor-phenyl)-imid nach Anspruch 1.

11. Verfahren zur Herstellung einer Verbindung der Formel I nach Anspruch 1, dadurch gekennzeichnet, dass man
a) eine Verbindung der Formel II

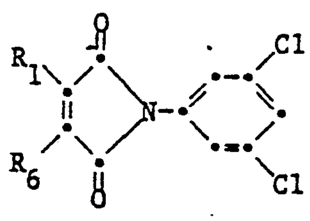

(II)

durch Lichteinwirkung bei einer Wellenlänge kürzer als 5000 Ångström mit einer Verbindung der Formel III

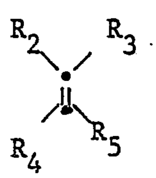

(III)

im Sinne einer photochemischen [2+2]-Cycloaddition umsetzt, oder
b) die Verbindung der Formel III schrittweise unter gleichen Reaktionsbedingungen einleitend mit einer Verbindung der Formel IV

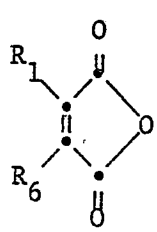

(IV)

zu einer Verbindung der Formel V

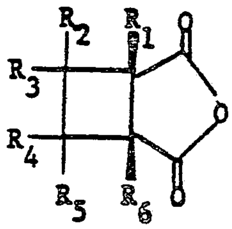

(V)

cycloaddiert, diese mit 3,5-Dichloranilin reagieren lässt und die dabei entstehende Amidcarbonsäure der Formel VI

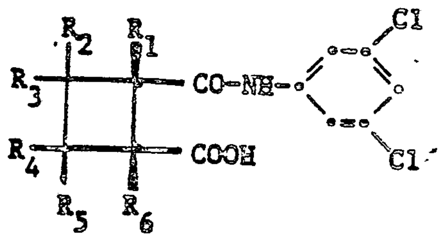

(VI)

anschliessend auf übliche Art unter Wasserabspaltung cyclisiert, wobei $R_1$ bis $R_6$ in den obigen Formeln II bis VI die unter Formel I angegebene Bedeutung haben.

12. Verfahren gemäss Anspruch 11, wobei die photochemische Cycloaddition in Gegegenwart eines Sensibilisators durchgeführt wird.

13. Verfahren gemäss Anspruch 12, wobei als Sensibilisator eine Verbindung verwendet wird, die eine Triplett-Energie von $\geq$230 kJ/Mol aufweist.

14. Verfahren gemäss einem der Ansprüche 11 oder 12, dadurch gekennzeichnet, dass man die Umsetzung einer Verbindung der Formel II mit einer Verbindung der Formel III bzw. die Umsetzung einer Verbindung der Formel IV mit einer Verbindung der Formel III in Gegenwart eines inerten organischen Lösungsmittels bei einer Temperatur zwischen -80°C und +30°C vornimmt.

15. Verfahren gemäss Anspruch 14, wobei die Reaktion im Temperaturbereich von -78°C bis -20°C durchgeführt wird.

16. Fungizide Mittel, enthaltend als Aktivsubstanz eine Verbindung der Formel I nach Anspruch 1 sowie geeignetes Trägermaterial und/oder Zuschlagstoffe.

17. Mittel nach Anspruch 16 enthaltend als Aktivsubstanz eine Verbindung der Formel I nach einem der Ansprüche 2 bis 10.

18. Verwendung einer Verbindung der Formel I nach Anspruch 1 zur Bekämpfung von Pilzen.

19. Verwendung nach Anspruch 18 einer Verbindung der Formel I nach einem der Ansprüche 2 bis 10.

20. Eine Verbindung der Formel V

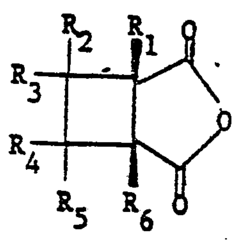

(V),

worin eines von $R_1$ und $R_6$ Wasserstoff, Methyl oder Aethyl und das andere Methyl oder Aethyl bedeutet, $R_2$ und $R_3$ unabhängig voneinander Wasserstoff, Fluor oder Methyl darstellen oder aber $R_2$ Acetoxy oder Chlor und gleichzeitig $R_3$ Wasserstoff bedeutet; $R_4$ Wasserstoff, Methyl oder Chlor ist und $R_5$ Wasserstoff oder Methyl darstellt, wobei auch $R_3$ und $R_4$ zusammen eine zusätzliche Bindung im Vierring bilden können.

21. Eine Verbindung der Formel V gemäss Anspruch 20, worin $R_1$ und $R_6$ gleiche Bedeutung haben und Methyl oder Aethyl darstellen, $R_2$ und $R_3$ unabhängig voneinander Wasserstoff, Fluor oder Methyl darstelle und $R_4$ und $R_5$ gleiche Bedeutung haben und Wasserstoff oder Methyl darstellen.